# FASCICULE DE BREVET EUROPEEN

(11) **EP 3 110 791 B1**
(45) Date de publication et mention de la délivrance du brevet: **18.04.2018**
(21) Numéro de dépôt: 15707361.0
(22) Date de dépôt: 02.03.2015
(51) Int. Cl.: C07C 269/04, C07C 227/04, C07D 263/06

(54) **PROCEDE DE PREPARATION DE DERIVES D'ACIDE 2,4-DIAMINO-3-HYDROXYBUTYRIQUE**
VERFAHREN ZUR HERSTELLUNG VON 2,4-DIAMINO-3-HYDROXYBUTTERSÄUREDERIVATE
PROCESS FOR PREPARING 2,4-DIAMINO-3-HYDROXYBUTYRIC ACID DERIVATIVES

(30) Priorité: 28.02.2014 FR 1451623
(43) Date de publication de la demande: 04.01.2017
(73) Titulaire: Nosopharm, 30000 Nimes (FR)
(72) Inventeur: RACINE, Emilie, F-30230 Bouillargues (FR)
(74) Mandataire: Regimbeau
(86) Numéro de dépôt international: PCT/EP2015/054248
(87) Numéro de publication internationale: WO 2015/128504

(56) Documents cités:
- WO-A2-01/38500
- ANTONIA STEPAN ET AL: "Stereoselective Synthesis of Orthogonally Protected [beta]-Hydroxy-[alpha]-,[gamma]-diamino Butyric Acids", SYNLETT, vol. 2011, no. 17, 27 septembre 2011 (2011-09-27), pages 2499-2504, XP055146945, ISSN: 0936-5214, DOI: 10.1055/s-0030-1260331 cité dans la demande
- SICHER J ET AL: "Amino Acids and Peptides XXVIII. (Stereospecific) synthesis of threo- and", COLLECTION OF CZECHOSLOVAK CHEMICAL COMMUNICATIONS, INSTITUTE OF ORGANIC CHEMISTRY & BIOCHEMISTRY, PRAGUE; CZ, vol. 24, 1 janvier 1959 (1959-01-01), pages 3719-3729, XP008172798, ISSN: 0010-0765, DOI: 10.1135/CCCC19593719 cité dans la demande
- R. REINER ET AL: "Zur Kenntnis des Muscazons. 24. Mitteilung �ber Inhaltsstoffe von Fliegenpilzen", HELVETICA CHIMICA ACTA, vol. 50, no. 1, 1 janvier 1967 (1967-01-01), pages 128-136, XP055146942, ISSN: 0018-019X, DOI: 10.1002/hlca.19670500120 cité dans la demande
- MARIANA L. GUTIERREZ ET AL: "Serine Hydroxymethyl Transferase fromStreptococcus thermophilus andL-Threonine Aldolase fromEscherichia coli as Stereocomplementary Biocatalysts for the Synthesis of [beta]-Hydroxy-[alpha],[omega]-diamino Acid Derivatives", CHEMISTRY - A EUROPEAN JOURNAL, vol. 14, no. 15, 19 mai 2008 (2008-05-19), pages 4647-4656, XP055146949, ISSN: 0947-6539, DOI: 10.1002/chem.200800031 cité dans la demande
- ALESSIA AMORE ET AL: "Development of a Hypersensitive Periodate-Cleavable Amino Acid that is Methionine- and Disulfide-Compatible and its Application in MHC Exchange Reagents for T Cell Characterisation", CHEMBIOCHEM, vol. 14, no. 1, 2 janvier 2013 (2013-01-02), pages 123-131, XP055147135, ISSN: 1439-4227, DOI: 10.1002/cbic.201200540

## Description

### DOMAINE DE L'INVENTION

La présente invention concerne un procédé de préparation de dérivés d'acide 2,4-diamino-3-hydroxybutyrique.

### ETAT DE LA TECHNIQUE

Les similitudes structurelles entre l'acide 2,4-diamino-butyrique, l'allothréonine et l'acide 2(S),4-diamino-3(S)-hydroxybutyrique rendent cette dernière molécule tout particulièrement attractive pour une utilisation dans l'industrie pharmaceutique, agrochimique ou en sciences des matériaux. L'acide 2(S),4-diamino-3(S)-hydroxybutyrique et ses dérivés protégés, tels que les acides 2(S),4-diamino-3(S)-hydroxybutyriques dont les fonctions amines primaires et éventuellement la fonction hydroxyle sont protégées, peuvent ainsi être utiles en tant que bloc de construction dans la synthèse de diverses molécules, en particulier dans la synthèse de nouveaux peptides. Il apparaît alors désirable de pouvoir synthétiser ces composés à grande échelle et à moindre coût.

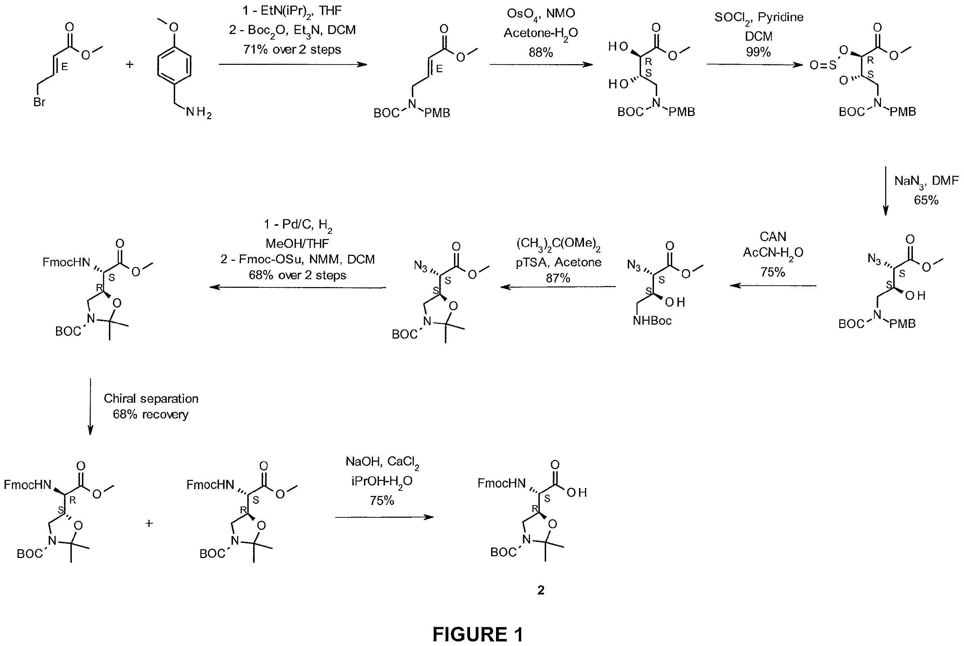

L'acide 2(S),4-diamino-3(S)-hydroxybutyrique n'est actuellement pas disponible dans le commerce rendant la synthèse de ses dérivés protégés difficile sur le plan industriel. Il a été proposé de préparer celui-ci en trois étapes à partir de la muscazone (1). Il a également été proposé de préparer l'acide 2,4-diamino-3-hydroxybutyrique racémique en neuf étapes à partir de dicarbométhoxypyrazoline (2). Récemment, il a été proposé de préparer les quatre diastéréoisomères de l'acide 2,4-diamino-3-hydroxybutyrique dont les fonctions amines primaires et hydroxyle sont protégées (désignés ici «acide 2,4-diamino-3-hydroxybutyrique protégé ») en dix étapes (figure 1) (3). La voie de synthèse proposée comprend, en avant-dernière étape, une séparation chirale conduisant à des pertes non négligeables en produit. Par ailleurs, cette synthèse, réalisée à petite échelle, n'a conduit qu'à quelques milligrammes du produit d'intérêt «acide 2,4-diamino-3-hydroxybutyrique protégé » avec un rendement molaire global ne dépassant pas les 6.4%. Une telle synthèse ne peut être réalistement mise en oeuvre à grande échelle.

D'autres équipes ont décrit la synthèse de précurseurs de l'acide 2(S),4-diamino-3(S)-hydroxybutyrique protégé. Par exemple, Wong et al. ont décrit la synthèse par voie biocatalytique de l'acide (hydroxyle azido) butyrique par réaction entre la glycine et un azidoacétaldéhyde en présence de L-thréonine aldolase (4). La stéréochimie du produit obtenu est dite (2S, 3S), cependant le ratio diastéréoisomérique n'est pas mentionné. Plus récemment, Clapés et al. ont décrit la réaction entre la glycine et un amino aldéhyde protégé en présence d'une sérine hydroxyméthyl transférase isolée de *Streptococcus thermophilus* (5). L'acide amino hydroxy butyrique protégé (2S, 3S) a été obtenu avec un rendement molaire de 13% et un ratio diastéréosiomérique modeste (86:14 , 2S, 3S : 2S, 3R). L'enzyme utilisée dans ce procédé n'étant pas disponible dans le commerce, elle doit être produite et purifiée suivant une méthode complexe rendant ces procédés difficiles à mettre en oeuvre à grande échelle. Rapoport et al. ont, quant à eux, préparés des précurseurs de l'acide 2(S),4-diamino-3(S)-hydroxybutyrique sous forme protégée lors de la synthèse totale de molécules complexes (6). L'époxydation de l'acide 2-amino-3-buténoïque, obtenu en trois étapes à partir de l'ester méthylique de la L-méthionine, a conduit à un mélange des deux diastéréoisomères (4:1, 2S, 3S : 2S, 3R). L'ouverture de l'époxyde 2S, 3S avec un ion azoture a conduit à un précurseur de l'acide 2(S),4-diamino-3(S)-hydroxybutyrique protégé. Cette méthode ne peut cependant être appliquée à grande échelle en raison de la faible stéréosélectivité de l'étape d'époxydation et du nombre élevé d'étapes nécessaires pour accéder au composé d'intérêt (8 étapes).

Un besoin demeure donc pour la mise au point d'un procédé de préparation de l'acide 2(S),4-diamino-3(S)-hydroxybutyrique protégé ou de précurseurs de celui-ci permettant leur obtention à grande échelle et à moindre coût.

### RESUME DE L'INVENTION

La présente invention porte sur un procédé de synthèse de composés de formule (I) suivante : 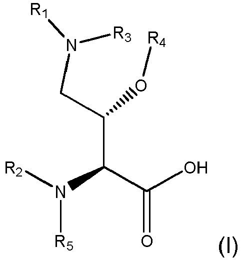 dans laquelle :
- R₁ et R₂ désignent, indépendamment l'un de l'autre, des groupements protecteurs des fonctions amines ;
- R₃ désigne un hydrogène ou un groupement protecteur des fonctions amines et R₄ désigne un hydrogène ou un groupement protecteur des fonctions hydroxyles, ou R₃ et R₄ forment ensemble un groupement choisi parmi -C(CH₃)₂-, -CH(CH₃)-, -C((CH₂)₄)-, -C((CH₂)₅)-, -CH(C₆H₅)-, -CH((*p*-OCH₃)C₆H₄)-, -CH((*m,p*-OCH₃)C₆H₃)- et -C(O)-;
- R₅ désigne un hydrogène, un radical alkyle, un radical aryle ou un radical hétéroaryle;
à partir de la 5-hydroxyectoïne.

La présente invention porte également sur un procédé de synthèse de l'acide 2,4-diamino-3(S)-hydroxycarboxylique à partir de la 5-hydroxyectoïne.

### FIGURE

La figure 1 représente le schéma de synthèse des quatre diastéréoisomères dérivés de l'acide 2,4-diamino-3-hydroxybutyrique dont les fonctions amines primaires et hydroxyle sont protégées selon l'art antérieur.

### DESCRIPTION DETAILLEE DE L'INVENTION

Les inventeurs ont mis au point un procédé de préparation de dérivés protégés de l'acide 2(S),4-diamino-3(S)-hydroxy butyrique, à partir de l'acide (4S, 5S)-5-hydroxy-2-méthyl-1,4,5,6-tétrahydropyrimidine-4-carboxylique (**2**), plus communément désigné « 5-hydroxyectoïne » :

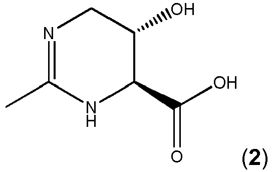

Par «dérivés protégés de l'acide 2(S),4-diamino-3(S)-hydroxy butyrique», est désigné dans la description de la présente invention un acide 2(S),4-diamino-3(S)-hydroxy butyrique dont les fonctions amines primaires, et éventuellement la fonction hydroxyle, sont protégées au moyen de groupements protecteurs adaptés. Les dérivés protégés de l'acide 2(S),4-diamino-3(S)-hydroxy butyrique peuvent être N-substitué en position 2. Les dérivés protégés de l'acide 2(S),4-diamino-3(S)-hydroxy butyrique sont représentés par la formule (I) suivante : 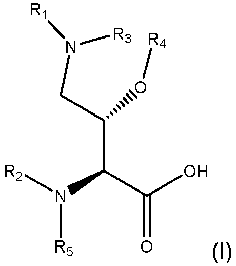 dans laquelle :
- R₁ et R₂ désignent, indépendamment l'un de l'autre, des groupements protecteurs des fonctions amines ;
- R₃ désigne un hydrogène ou un groupement protecteur des fonctions amines et R₄ désigne un hydrogène ou un groupement protecteur des fonctions hydroxyles, ou R₃ et R₄ forment ensemble un groupement choisi parmi -C(CH₃)₂-, -CH(CH₃)-, -C((CH₂)₄)-, -C((CH₂)₅)-, -CH(C₆H₅)-, -CH((*p*-OCH₃)C₆H₄)-, -CH((*m,p*-OCH₃)C₆H₃)- et -C(O)-;
- R₅ désigne un hydrogène, un radical alkyle, un radical aryle ou un radical hétéroaryle.

Dans la formule (I) ci-dessus, R₁ et R₂ peuvent être différents ou identiques. Lorsque les composés de formule (I) sont employés en synthèse peptidique, R₁ et R₂ sont de préférence différents.

Les groupements protecteurs des fonctions amines, primaires ou secondaires, sont bien connus de l'homme du métier. Ces groupements protègent les fonctions amines des réactions indésirables. Par exemple, une réaction chimique peut être effectuée sélectivement au niveau d'un autre site réactif qui est lui non protégé. Les groupements protecteurs des fonctions amines peuvent être tels que décrits dans "Protective Groups In Organic synthesis", (John Wiley & Sons, New York (1981)) et Harrison et al. « Compendium of Synthetic Organic Methods", Vols. 1 à 8 (J. Wiley & sons, 1971 à 1996). Les groupements protecteurs des fonctions amines comprennent les carbamates, amides, dérivés amino acétal, dérivés N-benzylé, dérivés imine, et dérivés N-hétéroatome. En particulier, R₁ et R₂ peuvent être choisis parmi l'acétyle, le benzoyle, le pivaloyle, le phénylsulfonyle, le benzyle (Bn), le t-butyloxycarbonyle (Boc), le benzyloxycarbonyle (Cbz), le p-méthoxybenzyloxycarbonyle, le p-nitrobenzyl-oxycarbonyle, le trichloroéthoxycarbonyl (TROC), l'allyloxycarbonyle (Alloc), le 9-Fluorénylméthyloxycarbonyl (Fmoc), le trifluoro-acétyle, les carbamates de benzyle (substitués ou non) et similaires. De manière préférée, dans la formule (I) ci-dessus, R₁ est un groupement Boc et R₂ est un groupement Fmoc.

Les groupements protecteurs des fonctions hydroxyles sont bien connus de l'homme du métier. Ces groupements protègent les fonctions hydroxyles des réactions indésirables. Les groupements protecteurs des fonctions hydroxyles peuvent être tels que décrits dans Greene, "Protective Groups In Organic synthesis", (John Wiley & Sons, New York (1981)) et Harrison et al. « Compendium of Synthetic Organic Methods", Vols. 1 à 8 (J. Wiley & sons, 1971 à 1996). Les groupements protecteurs des fonctions hydroxyles comprennent les éthers ou les esters de méthyle ou d'alkyle substitués ou non, par exemple, méthoxyméthyle, benzyloxyméthyle, 2-méthoxyéthoxyméthyle, 2-(triméthylsilyle) éthoxyméthyle, t-butyle, benzyle et triphénylméthyle, les éthers de benzyle (substitués ou non), les tétrahydropyranyle éthers, les éthers d'allyle, les éthyle éthers substitués, par exemple, 2,2,2-trichloroéthyle, les silyle éthers ou les éthers d'alkylsilyle, par exemple, triméthylsilyle, t-butyldiméthylsilyle et t-butyldiphénylsilyle, les éthers d'hétérocycle; et les esters préparés par réaction du groupe hydroxyle avec un acide carboxylique par exemple, les esters de tert-butyle, de benzyle ou de méthyle, les carbonates en particulier le carbonate de benzyle ou d'halogénoalkyle, l'acétate, le propionate, le benzoate et similaires.

Le terme « alkyle » désigne des chaînes hydrocarbonées saturées linéaires ou ramifiées comprenant de 1 à 20 atomes de carbone, de préférence de 1 à 12 atomes voire de 1 à 6 atomes de carbone. Des exemples de radical alkyle incluent les radicaux méthyle, éthyle, *n*-propyle, isopropyle, *n*-butyle, *iso*-butyle, *sec*-butyle, et *tert*-butyle.

Le terme « aryle » désigne un monocycle aromatique ou un système polycyclique comprenant au moins un cycle aromatique fusionné à au moins un autre cycle qui peut être aromatique ou non aromatique. Les radicaux aryles peuvent comprendre de 5 à 10 atomes de carbone. Le radical aryle peut être un phényle.

Le terme « hétéroaryle » désigne un aryle tel que défini ci-dessus dans lequel un ou plusieurs atomes de carbone est/sont remplacé(s) par un hétéroatome, tel qu'un atome d'azote, de soufre ou d'oxygène.

Les lettres « *p* » et « *m* » désignent respectivement les positions « para » et « méta » d'un radical phényle. Les lettres « *p,m* » indiquent que le radical phényle est substitué en position para et méta.

Sauf indications contraires, les réactions décrites ci-dessous sont conduites à pression ambiante et les rendements réactionnels indiqués sont des rendements molaires.

L'expression «température ambiante» désigne une température allant de 18°C à 25°C.

Les procédés mis au point par les inventeurs sont adaptés à une préparation à l'échelle industrielle des dérivés protégés de l'acide 2(S),4-diamino-3(S)-hydroxy butyrique par les nombreux avantages qu'ils présentent :
- les dérivés d'acide 2(S),4-diamino-3(S)-hydroxy butyrique sont obtenus en un nombre limité d'étapes à partir d'un réactif commercialement disponible et relativement peu onéreux, la 5-hydroxyectoïne ;
- la voie de synthèse ne nécessite pas de synthèse chirale car les centres chiraux sont présents dans le réactif de départ ;
- les conditions opératoires sont douces ;
- une seule étape de purification est nécessaire ;
- le produit est obtenu avec une pureté chirale élevée (dr>95 :5) ;
- le rendement global de la réaction conduisant aux composés de formule (I) dans laquelle R₁, R₂, R₃ et R₄ sont tels que définis ci-dessus et R₅ est un hydrogène est de l'ordre de 21%.

### Procédé de préparation des dérivés protégés de l'acide 2,4-diamino-3-hydroxy butyrique

Le procédé de préparation de composés de formule (I) suivante : 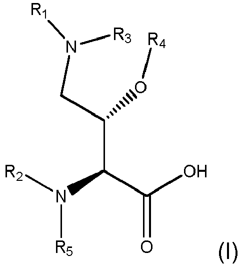 dans laquelle :
- R₁ et R₂ désignent, indépendamment l'un de l'autre, des groupements protecteurs des fonctions amines ;
- R₃ désigne un hydrogène ou un groupement protecteur des fonctions amines et R₄ désigne un hydrogène ou un groupement protecteur des fonctions hydroxyles, ou R₃ et R₄ forment ensemble un groupement choisi parmi -C(CH₃)₂-, -CH(CH₃)-, -C((CH₂)₄)-, -C((CH₂)₅)-, -CH(C₆H₅)-, -CH((*p*-OCH₃)C₆H₄)-, -CH((*m,p*-OCH₃)C₆H₃)- et -C(O)- ;
- R₅ désigne un hydrogène, un radical alkyle, un radical aryle ou un radical hétéroaryle ;
   comprend avantageusement les étapes suivantes :
   (a) hydrolyse basique de l'hydroxyectoïne et déacétylation pour conduire à l'acide 2,4-diamino-3(S)-hydroxy butyrique ;
   (b) protection régiosélective par un groupement R₁ de la fonction amine primaire en position 4 de l'acide 2,4-diamino-3(S)-hydroxybutyrique obtenu à l'étape (a) ;
   (c) protection par un groupement R₂ de la fonction amine primaire en position 2 de l'acide obtenu à l'étape (b) ;
   (d) éventuellement protection de la fonction hydroxyle en position 3 par un groupement R₄ et/ou protection de l'amine secondaire en position 4 par un groupement R₃ du composé obtenu à l'étape (c) ou protection des fonctions hydroxyle en position 3 et amine secondaire en position 4 pour obtenir un composé de formule (I) dans laquelle R₃ et R₄ forment ensemble un groupement choisi parmi -C(CH₃)₂-, -CH(CH₃)-, -C((CH₂)₄)-, -C((CH₂)₅)-, -CH(C₆H₅)-, -CH((*p*-OCH₃)C₆H₄)-, -CH((*m,p*-OCH₃)C₆H₃)- et -C(O)-;
   (e) éventuellement N-alkylation ou N-arylation en position 2 du composé obtenu à l'étape (d) pour obtenir un composé de formule (I) dans laquelle R₅ est un groupement alkyle ou un groupement aryle ou hétéroaryle ;
   (f) récupération du composé de formule (I) obtenu à l'étape (c) ou, le cas échéant, à l'étape (d) ou (e).

Les étapes (a), (b), (c), (d) et (e) peuvent être telles que décrites de manière détaillée ci-dessous.

### Hydrolyse basique de l'hydroxyectoïne et déacétylation (étape (a))

L'hydrolyse basique et la déacétylation peuvent être réalisées en une seule ou deux étapes pour conduire à l'acide 2,4-diamino-3(S)-hydroxy butyrique (**3**) :

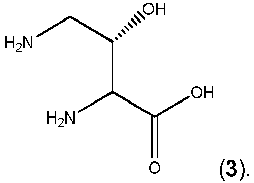

L'acide 2,4-diamino-3(S)-hydroxy butyrique est typiquement obtenu avec un ratio diastéréoisomérique (2S,3S : 2R,3S) d'au moins 70 :30. La conversion est quantitative (analyse par LC-MS).

### Hydrolyse et déacétylation en deux étapes

L'hydrolyse basique de l'hydroxyectoïne (ouverture du cycle de l'hydroxyectoïne par hydrolyse basique) peut être réalisée par chauffage à une température supérieure ou égale à 50°C pendant au moins 3h30 en présence d'au moins un équivalent molaire, de préférence d'un à deux équivalents, d'une base forte, telle que l'hydroxyde de sodium, l'hydroxide de potassium, l'hydroxide de lithium ou l'hydroxide de barium. De préférence, l'hydrolyse est réalisée en présence de deux équivalents molaires d'hydroxyde de sodium à une température de 50°C pendant cinq heures. L'hydrolyse basique est typiquement réalisée en milieu exclusivement aqueux.

L'hydrolyse basique dans de telles conditions conduit à l'ouverture du cycle de l'hydroxyectoïne produisant un dérivé monoacétylé. L'intermédiaire obtenu est alors déacétylé par hydrolyse acide, par exemple en présence d'acide chlorhydrique, d'acide sulfurique, d'acide nitrique, d'acide iodohydrique, d'acide fluorohydrique, d'acide perchlorique ou d'acide bromohydrique, par chauffage à une température d'au moins 95°C pendant au moins 1 heure. L'hydrolyse acide peut être conduite en une ou plusieurs étapes, en fonction des concentrations en jeu. Ainsi, au besoin, une première hydrolyse acide peut être réalisée, suivie d'une élimination de l'eau puis d'une deuxième hydrolyse.
La déacétylation est de préférence réalisée en présence d'acide chlorhydrique concentré 12 N à une température d'au moins 100°C pendant 1 heure. La déacétylation est typiquement réalisée directement sur le brut réactionnel obtenu à l'issue de l'étape d'hydrolyse basique.
Une hydrolyse basique réalisée en présence de deux équivalents molaires d'hydroxyde de sodium à une température de 50°C pendant cinq heures, suivie d'une déacétylation par hydrolyse acide en présence d'acide chlorhydrique concentré 12 N à une température d'au moins 100°C pendant 1 heure conduit à l'acide 2(S),4-diamino-3(S)-hydroxy butyrique avec un ratio diastéréoisomérique (2S,3S : 2R,3S) de 70 : 30.

### Hydrolyse et déacétylation en une étape

De manière alternative, l'hydrolyse basique de l'hydroxyectoïne (ouverture du cycle de l'hydroxyectoïne par hydrolyse basique) et la déacétylation peuvent être réalisées en une seule étape par chauffage à une température supérieure ou égale à 85°C pendant au moins 18h en présence d'au moins sept équivalents molaires d'une base forte. Les bases fortes peuvent être telles que décrites ci-dessus. La réaction est typiquement réalisée en milieu exclusivement aqueux.

L'hydrolyse basique de l'hydroxyectoïne (ouverture du cycle de l'hydroxyectoïne par hydrolyse basique) et la déacétylation permettent d'obtenir aisément l'acide 2,4-diamino-3(S)-hydroxycarboxylique. Ainsi dans un aspect, la présente invention porte également sur un procédé de synthèse de l'acide 2,4-diamino-3(S)-hydroxycarboxylique à partir de la 5-hydroxyectoïne. Le procédé comprend une hydrolyse basique de l'hydroxyectoïne (ouverture du cycle de l'hydroxyectoïne par hydrolyse basique) et une déacétylation pour conduire à l'acide 2,4-diamino-3(S)-hydroxy butyrique. L'hydrolyse basique et la déacétylation sont telles que décrites ci-dessus. L'acide 2,4-diamino-3(S)-hydroxycarboxylique est obtenu sous forme de mélange de diastéréoisomères : l'acide 2(S),4-diamino-3(S)-hydroxycarboxylique et l'acide 2(R),4-diamino-3(S)-hydroxycarboxylique. Les composés obtenus peuvent être séparés par des méthodes bien connues de l'homme du métier, telles que par chromatographie.

### Protection régiosélective de la fonction amine primaire en position 4 (étape (b))

La protection régiosélective de la fonction amine primaire en position 4 de l'acide 2,4-diamino-3(S)-hydroxybutyrique (**3**) obtenu à l'étape (a) peut être réalisée de différentes manières adaptées. Elle conduit aux composés de formule (I) dans laquelle R₂, R₃, R₄ et R₅ représentent des atomes d'hydrogène et R₁ représente un groupement protecteur des fonctions amines, soit aux composés de formule (la) : 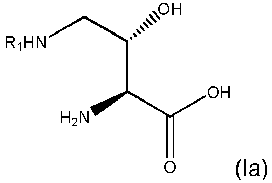 dans laquelle R₁ est tel que défini ci-dessus.

Dans certains modes de réalisation, la protection régiosélective peut être réalisée en trois étapes.
Dans un premier temps, un complexe de cuivre est préparé par mise en présence de l'acide 2,4-diamino-3(S)-hydroxybutyrique obtenu à l'étape (a) avec un complexe de cuivre, tel que CuSO₄.5H₂O, CuSO₄, Cu₂(OH)₂CO₃, Cu(OAc)₂ ou CuCO₃. La réaction est typiquement réalisée dans l'eau, à reflux ou température ambiante. La complexation permet de protéger simultanément la fonction carbonyle et la fonction amine primaire en position 2 du composé obtenu à l'étape (a). De préférence, un complexe de cuivre est préparé au moyen de sulfate de cuivre pentahydraté.
Dans un second temps, la fonction amine primaire en position 4 est protégée au moyen d'un groupement protecteur R₁ adapté. Par exemple, la fonction amine primaire en position 4 peut être protégée par un groupement t-butoxycarbonyle (Boc) ou benzyloxycarbonyle, p-méthoxybenzyloxycarbonyle, le p-nitrobenzyl-oxycarbonyle, le trichloroéthoxycarbonyl (TROC), l'allyloxycarbonyle (Alloc), le 9-Fluorénylméthyloxycarbonyl (Fmoc). La protection par de tels groupes protecteurs peut être réalisée dans des conditions bien connues de l'homme du métier. Typiquement, la réaction est réalisée à température ambiante. De préférence, R₁ est un groupement Boc. L'introduction d'un groupement protecteur Boc est de préférence réalisée en faisant réagir le composé obtenu après complexation avec du cuivre avec un anhydre de formule (Boc)₂O ou N₃CO₂tBu/MgO dans un solvant, tel que l'acétone, l'eau, le méthanol, l'éthanol, le THF ou le dioxane.
Dans un troisième temps, le cuivre est décomplexé. La décomplexation du cuivre peut être réalisée par réaction avec du 8-quinolinol ou des résines décomplexantes disponibles commercialement (Chelex 100 par exemple) ou des sels d'EDTA. Typiquement, la réaction est réalisée à température ambiante. De préférence, la décomplexation du cuivre est réalisée avec du 8-quinolinol, typiquement dans l'eau. Cette troisième étape de décomplexation du cuivre est optionnelle. Ainsi, il est également possible d'utiliser dans la suite du procédé directement le complexe de cuivre obtenu.
De manière surprenante, l'acide 2,4-diamino-3-hydroxybutyrique protégé en position 4 de configuration (2S, 3S), ou son complexe de cuivre, est obtenu à l'issue de ces étapes avec une pureté diastéréoisomérique supérieure à 90%, de préférence supérieure à 95%.

De manière alternative, la protection régiosélective de la fonction amine primaire en position 4 de l'acide 2,4-diamino-3(S)-hydroxybutyrique obtenu à l'étape (a) peut être réalisée par complexation de l'atome de bore du 9-BBN par la fonction amine en position 2 et la fonction acide. Cette complexation permet la protection temporaire de la fonction amine en position 2. Dans un second temps, la fonction amine primaire en position 4 est protégée au moyen d'un groupement protecteur Boc. Les conditions réactionnelles de la réaction de protection sont bien connues de l'homme du métier. Typiquement, la réaction est réalisée à température ambiante. Par exemple par la fonction amine primaire en position 4 est protégée au moyen d'un groupement protecteur Boc par réaction du complexe obtenu, en conditions basiques, avec Boc₂O. Le bore est ensuite décomplexé en présence d'éthylène diamine.

Dans certains modes de réalisation, la protection régiosélective de la fonction amine primaire en position 4 de l'acide 2,4-diamino-3(S)-hydroxybutyrique obtenu à l'étape (a) peut être réalisée par réaction avec de l'hydroxyde de sodium et (Boc)₂O ou N₃CO₂ ou encore avec du 1H-benzotriazole, du DMAP et Boc₂O ou PhOCO₂tBu, typiquement à température ambiante.

Lorsque la protection régiosélective de la fonction amine primaire en position 4 est réalisée par un groupement Boc, le composé (**4**) suivant, ou son complexe de cuivre (4)₂Cu, est obtenu :

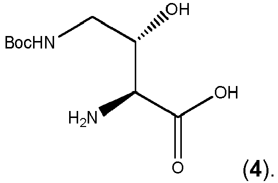

### Protection de la fonction amine primaire en position 2 (étape (c))

La fonction amine primaire en position 2 est protégée au moyen d'un groupement protecteur R₂ adapté. Cette étape conduit aux composés de formule (I) dans laquelle R₃, R₄ et R₅ représentent des atomes d'hydrogène, soit aux composés de formule (Ib) suivante : 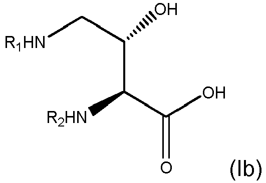 dans laquelle R₁ et R₂ sont tels que définis ci-dessus.

Le groupement R₂ peut être un groupement t-butoxycarbonyle (Boc), benzyloxycarbonyle, p-méthoxybenzyloxycarbonyle, p-nitrobenzyl-oxycarbonyle, trichloroéthoxycarbonyl (TROC), allyloxycarbonyle (Alloc) ou 9-Fluorénylméthyloxycarbonyl (Fmoc).
La protection de la fonction amine primaire en position 2 est de préférence réalisée par un groupement 9-fluorényl-méthoxycarbonyl (Fmoc). L'introduction d'un groupement Fmoc peut être réalisée dans des conditions bien connues de l'homme du métier, par exemple, par réaction du produit obtenu à l'étape (b) avec le 9-fluorényl-méthoxycarbonyl hydroxy succinimide (FmocOSu) en présence d'un solvant tel que du dioxane, typiquement à température ambiante.
Lorsque que R₁ représente un groupement Boc et R₂ un groupement Fmoc, le composé (**5**) suivant est obtenu :

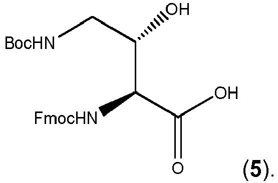

Le composé de formule (Ib) obtenu à l'issue de cette étape peut être récupéré. Il peut être purifié par chromatographie sur colonne de silice avec un gradient Heptane/acétate d'éthyle de 100 :0 à 0 :100 (volume : volume). Le produit est alors obtenu avec un rendement de 30% sur les quatre premières étapes.
Une pureté diastéréoisomérique supérieure à 90%, de préférence supérieure à 95%, est obtenue.

Le composé de formule (Ib) obtenu à l'issue de cette étape (c) peut éventuellement être soumis aux étapes (d) et éventuellement (e) décrites ci-dessous, avant ou après purification.

### Protection des fonctions hydroxyle en position 3 et amine secondaire en position 4 (étape (d))

La protection de la fonction hydroxyle en position 3 et/ou de la fonction amine secondaire en position 4 peut être réalisée au moyen de groupements protecteurs adaptés conduisant aux composés de formule (I) dans laquelle R₁ et R₂ sont tels que décrits ci-dessus, R₅ désigne un hydrogène, R₃ désigne un hydrogène ou un groupement protecteur des fonctions amines et R₄ désigne un hydrogène ou un groupement protecteur des fonctions hydroxyles, ou R₃ et R₄ forment ensemble un groupement choisi parmi -C(CH₃)₂, -CH(CH₃)-, -C((CH₂)₄)-, -C((CH₂)₅)-, -CH(C₆H₅)-, -CH((*p*-OCH₃)C₆H₄)-, -CH((*m,p*-OCH₃)C₆H₃)- et -C(O)-, soit des composés de formule (Ic) suivante :

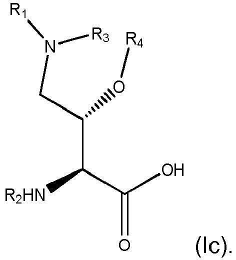

Dans certains modes de réalisation, la protection de la fonction hydroxyle en position 3 et de la fonction amine secondaire en position 4 est réalisée au moyen d'un isopropylidène conduisant aux composés de formule (I) telle que décrite ci-dessus dans laquelle R₃ et R₄ forment ensemble un groupement-C(CH₃)₂-, soit aux composés de formule (Id) suivante : 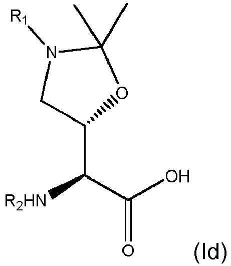 dans laquelle R₁ et R₂ sont tels que décrits ci-dessus.

La réaction entre le composé de formule (Ib) obtenu à l'issue de l'étape (c) et le 2,2'-diméthoxy propane est typiquement réalisée dans du dichlorométhane en présence de BF₃.OEt₂, de préférence à 0°C.
Lorsque que R₁ représente un groupement Boc et R₂ un groupement Fmoc, le composé (1) suivant est obtenu :

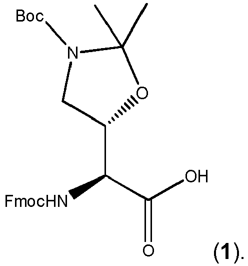

Le composé est obtenu à l'issue de cette étape avec une pureté diastéréoisomérique supérieure à 90%, de préférence supérieure à 95%. Le rendement de la réaction est de 70%. Le composé obtenu peut ensuite être purifié par recristallisation.

### N-alkylation ou N-arylation en position 2 (étape (e))

La synthèse de dérivés *N*-substitués peut être réalisée en trois étapes à partir du composé obtenu à l'étape (d) : estérification de la fonction acide, N-substitution (N-alkylation ou N-arylation) de la fonction carbamate libre et saponification chimio sélective de la fonction ester en présence du groupement Fmoc.
Les réactions sont réalisées dans des conditions bien connues de l'homme du métier. La première étape d'estérification se déroule en présence d'un alcool et d'un agent de couplage. La N-substitution sur la fonction carbamate libre peut être de deux natures :
- N-alkylation en présence d'un agent alkylant RX, tel qu'un halogénure d'alkyle, un sulfonate d'alkyle, éventuellement d'une base (différente d'une amine secondaire) et éventuellement d'un solvant (différent d'une amine secondaire et d'un alcool).
- N-arylation en présence d'un cycle aromatique ou hétéroaromatique portant une fonction lui permettant de réagir dans des couplages catalysés par des métaux (halogénures, sulfonate, diazonium..) et d'un système catalyseur (un métal avec éventuellement un ligand, éventuellement une base et éventuellement un solvant).
La saponification de la fonction ester en présence du groupement protecteur Fmoc est réalisée de manière sélective, de préférence en présence de NaOH et de CaCl₂, ou en présence de Lil ou de Me₃SnOH.

En particulier, les dérivés *N*-substitués peuvent être obtenus selon le schéma de synthèse suivant :

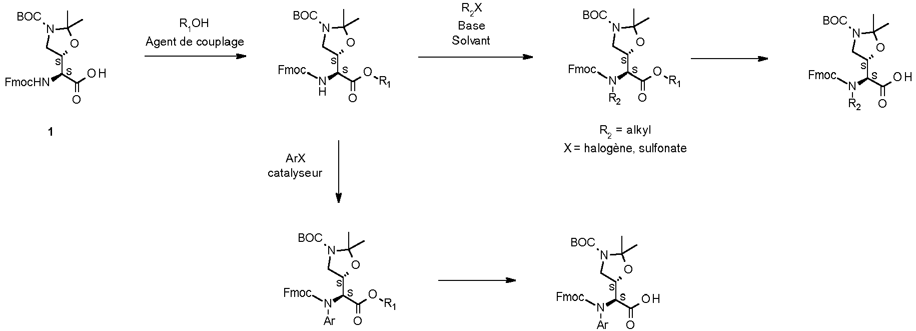

Les composés obtenus à l'issue de l'étape (d) et éventuellement (e) sont récupérés. Ils peuvent être purifiés par des méthodes bien connues de l'homme du métier.

Dans un mode de mise en oeuvre de la présente invention, des dérivés protégés de l'acide 2,4-diamino-3-hydroxy butyrique sont préparés selon le schéma de synthèse suivant :

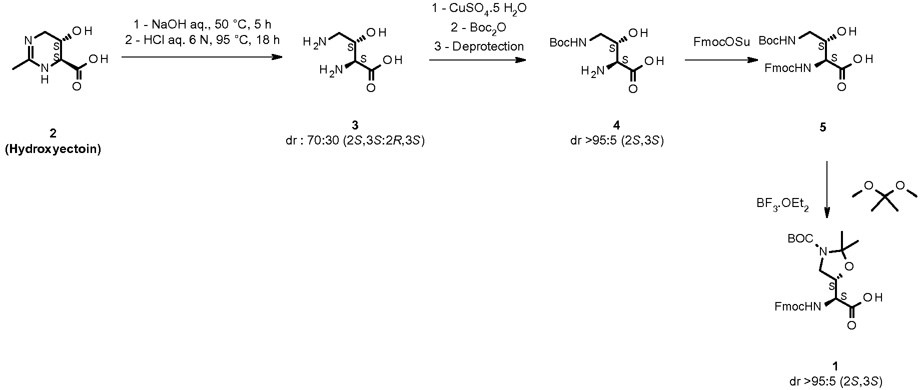

Le produit désiré (1) est obtenu avec un rendement global de l'ordre de 21% et une pureté chirale supérieure à 95%.

### EXEMPLES

### Exemple 1 :

### Préparation du composé (3)

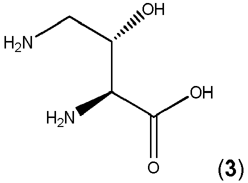

10 g d'hydroxyectoïne (63,2 mmol) sont dissouts dans 200 mL d'eau. 5.1 g d'hydroxyde de sodium (2,0 équiv., 126,6 mmol) sont ajoutés. La solution est agitée à 50 ° C pendant 5 heures.

Une analyse par LC-MS du mélange réactionnel a montré la disparition du réactif de départ et l'apparition d'un nouveau composé ([M+H⁺]=177) correspondant à un intermédiaire mono acétylé.

La solution est refroidie à 0 °C. De l'acide chlorhydrique concentré est alors ajouté jusqu'à l'obtention d'un pH de 1 (∼ 50 ml). La solution est ensuite chauffée à 95 ° C pendant 18 heures.

Une analyse par LC-MS du mélange réactionnel a montré la disparition de l'intermédiaire mono acétylé et la formation de l'acide 2,4-diamino-3-hydroxy butyrique **3** ([M+ H⁺] = 135).

La solution est concentrée pour donner un solide jaune pâle (20,8 g).

L'analyse de Marfey de cet échantillon a montré qu'il s'agit d'un mélange de deux diastéréoisomères (2S, 3S/2R, 3S = 70:30).

### Préparation du composé (4)

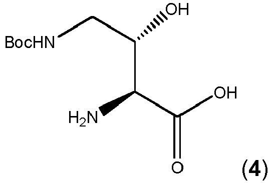

Le solide jaune pâle obtenu à l'étape précédente est dissout dans 60 mL d'eau. La solution obtenue est refroidi à 0°C. Du bicarbonate de sodium (2.0 équiv., 10.6 g, 126.4 mmol) est alors ajouté par portion. Une solution de CuSO₄.5 H₂O (0.5 équiv., 7.9 g, 31.6 mmol) dans de l'eau (20 mL) est ajoutée au goutte à goutte à la solution précédente. La solution verte obtenue est agitée à température ambiante pendant 18 heures. La solution est ensuite refroidie à 0 °C. Du bicarbonate de sodium (2.0 équiv., 10.6 g, 126.4 mmol) est alors ajouté par portion. Une fois que la solution a atteint la température ambiante, une solution de di -tert -butyle dicarbonate (1.3 éq., 17.9 g, 82.2 mmol) dans de l'acétone (20 mL) est ajoutée au goutte à goutte. La solution épaisse verte obtenue est agitée à température ambiante pendant 18 heures. 100 mL de méthanol sont alors ajoutés et le mélange est agité pendant 6 heures. La suspension bleue obtenue est filtrée. Un solide bleu est collecté (15.2 g).

Le solide est mis en suspension dans 500 mL d'eau. 22 g de 8 - quinolinol (2.5 équiv., 22.0 g, 156.0 mmol) sont ajoutés. Le mélange est agité à température ambiante pendant 18 heures et filtré. Le solide collecté est rincé à l'eau (50 mL). Les eaux collectées ont été analysées par LC- MS. La formation d'un nouveau composé est mise en évidence ([M+H⁺] = 235).

La solution est concentrée sous vide pour donner le composé **4** sous forme de solide brun pâle (30.1 g).

L'analyse de Marfey de cet échantillon a révélé la présence d'un seul diastéréoisomère (2S, 3S , dr > 95:5).

### Préparation du composé (5)

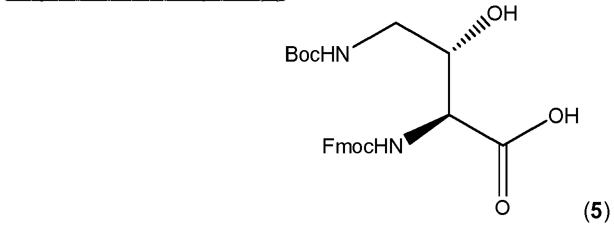

30,1 g du composé obtenu à l'étape précédente sont dissouts dans 1.5 L d'eau. La solution est refroidie à 0 °C. Du bicarbonate de sodium (2.0 équiv., 10.5 g, 125.6 mmol) est ajouté doucement. Une fois que la solution a atteint la température ambiante, une solution de FmocOSu (1.1 équiv., 23.2 g, 69.0 mmol) dans du dioxane (200 ml) est ajoutée au goutte à goutte sous agitation rapide. Un solide blanc est obtenu. La suspension est agitée à température ambiante pendant 18 heures. A l'issue de ce temps, la suspension est filtrée et le filtrat est concentré sous vide pour donner le composé sous forme de solide brun pâle (22.0 g) (LC-MS :[M+H⁺] = 457).

Le composé est purifié par chromatographie sur colonne de silice (Heptane/acétate d'éthyle de 100 :0 à 0 :100.). Le composé **5** est obtenu sous forme de solide blanc (8,6 g, pureté : 95% par LC-MS).

Le composé **5** est obtenu avec un rendement global de 30% sur 4 étapes.

### Préparation du composé (1)

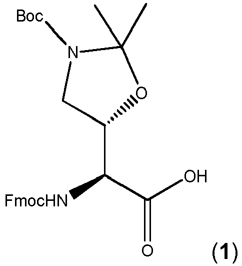

Le composé **5** (8,5 g, 18,6 mmol) est dissout dans du dichlorométhane (300 mL) et du 2,2'-diméthoxy propane (300 ml) est alors ajouté. La solution est refroidie à 0°C. 2 mL de BF₃.OEt₂ (cat.) sont alors ajoutés au goutte à goutte. La solution est laissée à température ambiante et agitée pendant 18 heures.

Le mélange réactionnel est ensuite lavé avec une solution aqueuse saturée en bicarbonate de sodium. Les phases aqueuses et organiques sont séparées. La phase organique est séchée sur sulfate de magnésium, filtrée et concentrée sous vide pour donner une huile jaune pâle. Cette huile a été triturée avec de l'heptane pour donner le composé **1** sous forme de solide blanc (6.5 g, pureté de 95% par LC-MS, 70% de rendement).

L'analyse de Marfey de cet échantillon révèle la présence du diastéréoisomère attendu (2S, 3S, dr> 95:5).

Le rendement global sur les 5 étapes de cette synthèse est de 21%.

### Exemple 2 :

Le procédé selon la présente invention peut également être appliqué en présence de quantités plus importantes de réactifs.

### Préparation du composé (3)

De l'hydroxyectoïne (50.0 g, 316.4 mmol) a été dissoute dans de l'eau (260 mL). NaOH (2.0 éq., 632.9 mmol, 25.3 g) a été ajouté par portion et le mélange a été agité à température ambiante jusqu'à dissolution totale du NaOH. La solution obtenue a été chauffée à 50 °C pendant 6 h puis refroidie à température ambiante puis à 5 °C avec un bain de glace. HCl aqueux (6 N) a été additionné lentement (-100 mL) jusqu'à obtenir une solution de pH = 4. La solution obtenue a été congelée à -80 °C puis lyophilisée. Le solide blanc obtenu a été dissout dans HCl aqueux (6 N, 300 mL) et le mélange a été chauffé à 110 °C pendant 3 heures (l'analyse par LC-MS montre le produit attendu et quelques impuretés). La solution obtenue a été diluée avec de l'eau (300 mL), congelée à -80 °C puis lyophilisée pour donner (3) sous la forme d'un solide jaune pâle (107 g, contient 2.0 éq. de NaCl, pureté >90% par LC-MS, l'analyse de Marfey a montré un mélange de deux diastéréoisomères (2S,3S/2R,3S = 70:30).
LC-MS: Rt = 2.30 min, [M+H]⁺ = 135
¹H NMR (D₂O, 600 MHz, mélange de 2 diastéréoisomères 70:30): d 3.22 (dd, J = 10.2 et 13.2 Hz, 0.3 H); 3.34-3.47 (m, 1.7H); 4.08 (d, J = 4.8 Hz, 0.3H); 4.24 (d, J = 3.0 Hz, 0.7H); 4.46 (td, J = 3.0 et 10.2 Hz, 0.7H); 4.48-4.52 (m, 0.3H).
¹³C NMR (D₂O, 150 MHz, mélange de 2 diastéréoisomères):d 42.65; 43.38; 57.70; 66.92; 67.45; 170.45.
Analyse de Marfey : 2S,3S/2R,3S = 73:27 (2S,3S : Rt = 96.01 min, 73%; 2R,3S: Rt = 97.84 min, 27%)

### Préparation du composé (4)₂Cu

Le mélange des deux diastéréoisomères obtenu à l'étape précédente pour la préparation du (2S,3S)-2,4-diamino-3-hydroxy-butanoic acid (3) (53 g, -160 mmol) a été mis dans un ballon de 2 L et a été dissout dans de l'eau (250 mL). NaOH (3.0 éq., 480 mmol, 19.0 g) a été ajouté par portion. Le mélange a été agité jusqu'à dissolution des solides et une solution de CuSO₄.5H₂O (0.5 éq., 80 mmol, 20.0 g) dans l'eau (125 mL) a été ajoutée lentement. La solution bleue foncée obtenue a été placée dans un bain d'huile à température ambiante. Le système a été chauffé de 25 à 110 °C (en 25 minutes) puis à 110 °C pendant 30 minutes puis refroidit à température ambiante pendant 4 heures. Une solution de Boc₂O (2.0 éq., 320 mmol, 52.0 g) dans le dioxane (275 mL) a été ajoutée et la réaction a été agitée à température ambiante pendant 70 heures. Une solution de Boc₂O (0.5 éq., 80 mmol, 13.0 g) dans le dioxane (60 mL) a été ajoutée lentement et le mélange agité à température ambiante pendant 24 heures. La suspension obtenue a été filtrée. Le solide bleu pâle obtenu a été rincé avec de l'eau (-700 mL), de l'éther diéthylique (-300 mL) puis séché pour donner du ((2S,3S)-2-amino-4-(tert-butoxycarbonylamino)-3-hydroxy-butanoic acid)₂Cu sous forme d'un solide bleu pâle (17.1 g, 40% de rendement sur deux étapes).

### Préparation du composé (5)

Ensuite, le ((2S,3S)-2-amino-4-(tert-butoxycarbonylamino)-3-hydroxy-butanoic acid)₂Cu précédemment obtenu (17.1 g, 32.0 mmol) a été suspendu dans l'eau (300 mL). Une solution de Na₂EDTA (1.5 éq., 48.0 mmol, 15.9 g) et de NaOH (3.0 éq., 96.0 mmol, 3.84 g) dans l'eau (300 mL) a été additionnée. Le mélange a été agité à température ambiante pendant 4 heures jusqu'à dissolution complète de la suspension. La solution obtenue a été refroidie dans un bain de glace puis une solution de FmocOSu (2.5 éq., 80.0 mmol, 35.7 g) dans le dioxane (500 mL) a été ajoutée doucement. A la fin de l'addition, Na₂CO₃ (2.5 éq., 80.0 mmol, 8.5 g) a été ajouté et le mélange a été réchauffé à température ambiante pendant 18 heures. La solution bleue limpide obtenue a été lavée avec de l'éther diéthylique (4*200 mL) puis refroidie dans un bain de glace. 1 N HCl aqueux a été ajouté lentement jusqu'à obtenir une solution à pH = 3-4 (-250 mL). Cette phase aqueuse a été extraite avec de l'acétate d'éthyle (5*200 mL). Les phases organiques ont été combinées, lavées avec une solution aqueuse saturée de NaCl (2*150 mL), séchées sur MgSO₄ filtrées et concentrées pour donner une huile jaune pâle. De l'acétonitrile (200 mL) a été ajouté et le mélange a été agité à température ambiante 70 heures. La suspension a été filtrée, le solide rincé avec de l'acétonitrile (100 mL) puis séché pour donner (**5**) sous forme de poudre blanche (29.1 g, rendement quantitatif, 95% de pureté par LC-MS). L'analyse de Marfey de cet échantillon a révélé la présence d'un seul diastéréoisomère (2S, 3S , dr > 95:5).
LC-MS: Rt = 13.96 min, 95% (254 nm), [M+H-Boc]⁺ = 357
¹H NMR (DMSO-d₆, 600 MHz, 343 K): d 1.39 (s, 9H); 3.00-3.04 (m, 1H); 3.12-3.20 (m, 1H); 3.85-3.88 (m, 1H); 4.00-4.13 (m, 1H); 4.22-4.25 (m, 1H); 4.28-4.31 (m, 2H); 6.61 (br s, 0.8H); 7.33 (t, J = 7.2 Hz, 2H); 7.42 (t, J = 7.2 Hz, 2H); 7.71 (d, J = 7.2 Hz, 2H); 7.87 (d, J = 7.2 Hz, 2H).
¹³C NMR (DMSO-d₆, 150 MHz, 343 K):d 27.93; 42.88; 46.48; 57.26; 65.69; 69.96; 77.56; 119.65; 124.87; 126.70; 127.24; 140.40; 143.50; 143.54; 151.30; 155.41; 155.65; 171.06.
Analyse de Marfey: Rt = 95.60 min (2S, 3S), 100% (340 nm), [M+H]⁺ = 695.15

### Préparation du composé (1)

Du (2S,3S)-4-(tert-butoxycarbonylamino)-2-(9H-fluoren-9-ylmethoxycarbonylamino)-3-hydroxy-butanoic acid (**5**) (29.1 g, 63.6 mmol) a été suspendu dans un mélange d'acétone et de 2,2-diméthoxypropane (1:1, 480 mL). La suspension a été refroidie avec un bain de glace et BF₃.OEt₂ (catalytique, 900 µL) a été additionné goutte à goutte. La réaction a été agitée dans le bain de glace jusqu'à obtenir une solution orange/marron. Une solution aqueuse saturée de NaHCO₃ (200 mL). La phase aqueuse a été extraite avec de l'acétate d'éthyle (2*200 mL). Les phases organiques ont été combinées, lavées avec une solution aqueuse 0.1 N HCl (200 mL) et une solution aqueuse saturée de NaCl (200 mL), séchées sur MgSO₄, filtrées et concentrées. L'huile jaune pâle obtenue a été dissoute dans de l'éther diéthylique (100 mL) et la solution a été refroidie avec un bain de glace puis de l'hexane (400 mL) a été ajouté. Lors de l'addition de l'hexane, la formation de solides a été observée. A la fin de l'addition, la présence d'un solide collant au fond du ballon a été observée. De l'éther diéthylique a été additionné à température ambiante et le mélange a été trituré jusqu'à obtenir un solide blanc qui a ensuite été trituré pendant 18 heures. La suspension obtenue a été filtrée pour donner (**1**) sous la forme d'une poudre blanche (22.9 g, 73% de rendement, 96% de pureté par LC-MS).
LC-MS : Rt = 19.66 min, 96% (254 nm), [M+H-Boc-CH(CH₃)₂]⁺ = 357
¹H NMR (DMSO-d₆, 600 MHz, 343 K) d 1.43 (s, 12H), 1.47 (s, 3H), 3.36-3.41 (m, 1H), 3.54-3.59 (m, 1H), 4.22-4.25 (m, 2H), 4.30-4.33 (m, 2H), 4.38 (br s, 1H), 7.30-7.34 (m, 2H), 7.39-7.44 (m, 2H), 7.58 (br s, 1H), 7.69-7.72 (m, 2H), 7.87 (d, J = 7.8, 2H).
¹³C NMR (DMSO-d₆, 150 MHz, 343 K):d 27.78, 46.48, 46.84, 65.72, 72.81, 78.86, 92.94, 119.65, 124.83, 126.66, 127.25, 140.41, 143.48, 151.04, 155.51, 170.37.
La régiosélectivité des protections a été déterminée grâce à des analyses RMN (HMBC et HSQC). Un signal clair a été observé en HMBC entre le CHa (4.25 ppm) et le CO du Fmoc protégeant l'amine en position a (155.5 ppm) prouvant ainsi la régiosélectivité des protections.
Analyse de Marfey: Rt = 96.19 min (2S, 3S), 100% (340 nm), [M+H]⁺ = 695.15

### REFERENCES

1. Reiner, R. ; Eugster, C. H. Helv. Chim. Acta 1967, 50, 128.
2. Sicher, J.; Rajsner, M.; Rudinger, J ; Eckstein, M.; Sorm, F. Coll. Czech. Chem. Comm; 1959, 24, 3719.
3. Stepan, A. F.; Nguyen, T.-T.; Anderson, D.; Liang, H.; Zhanshan, Q.; Magee, T. V. Synlett 2011, 2499.
4. Vassilev, V. P.; Uchiyama, T.; Kajimoto, T.; Wong, C.-H. Tetrahedron Lett. 1995, 36, 4081.
5. Vidal, L. ; Calveras, J. ; Clapés, P. ; Ferrer, P. ; Caminal, G. Appl. Microbiol. Biotechnol. 2005, 68, 489.
6. Shaw, K. J. ; Luly, J. R. ; Rapoport, H. J. Org. Chem. 1985, 50, 4515.

## Revendications

1. Procédé de synthèse de composés de formule (I) suivante : 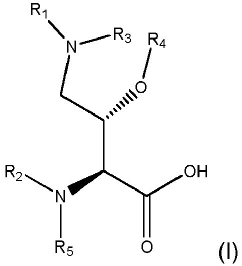 dans laquelle :
- R₁ et R₂ désignent, indépendamment l'un de l'autre, des groupements protecteurs des fonctions amines ;
- R₃ désigne un hydrogène ou un groupement protecteur des fonctions amines et R₄ désigne un hydrogène ou un groupement protecteur des fonctions hydroxyles, ou R₃ et R₄ forment ensemble un groupement choisi parmi -C(CH₃)₂-, -CH(CH₃)-, -C((CH₂)₄)-, -C((CH₂)₅)-, -CH(C₆H₅)-, -CH((*p*-OCH₃)C₆H₄)-, -CH((*m,p*-OCH₃)C₆H₃)- et -C(O)- ;
- R₅ désigne un hydrogène, un radical alkyle, un radical aryle ou un radical hétéroaryle ;
à partir de la 5-hydroxyectoïne.

2. Procédé de synthèse selon la revendication 1 comprenant les étapes suivantes :
(a) hydrolyse basique de l'hydroxyectoïne et déacétylation pour conduire à l'acide 2,4-diamino-3(S)-hydroxy butyrique ;
(b) protection régiosélective par un groupement R₁ de la fonction amine primaire en position 4 de l'acide 2,4-diamino-3(S)-hydroxybutyrique obtenu à l'étape (a) ;
(c) protection par un groupement R₂ de la fonction amine primaire en position 2 de l'acide obtenu à l'étape (b) ;
(d) éventuellement protection de la fonction hydroxyle en position 3 par un groupement R₄ et/ou protection de l'amine secondaire en position 4 par un groupement R₃ du composé obtenu à l'étape (c) ou protection des fonctions hydroxyle en position 3 et amine secondaire en position 4 pour obtenir un composé de formule (I) dans laquelle R₃ et R₄ forment ensemble un groupement choisi parmi -C(CH₃)₂-, -CH(CH₃)-, -C((CH₂)₄)-, -C((CH₂)₅)-, - CH(C₆H₅)-, -CH((*p*-OCH₃)C₆H₄)-, -CH((*m,p*-OCH₃)C₆H₃)- et -C(O)- ;
(e) éventuellement N-alkylation ou N-arylation en position 2 du composé obtenu à l'étape (d) pour obtenir un composé de formule (I) dans laquelle R₅ est un radical alkyle, un radical aryle ou un radical hétéroaryle;
(f) récupération du composé de formule (I) obtenu à l'étape (c) ou, le cas échéant, à l'étape (d) ou (e).

3. Procédé de synthèse selon la revendication 2 dans lequel l'hydrolyse et la déacétylation selon l'étape (a) sont réalisés en présence d'au moins 7 équivalents molaires d'une base forte pendant au moins 18h à une température supérieure ou égale à 85 °C.

4. Procédé de synthèse selon l'une des revendications précédentes dans lequel l'hydrolyse selon l'étape (a) est réalisée en présence d'au moins un équivalent molaire d'une base forte pendant au moins 3h30 à une température supérieure ou égale à 50 °C suivi d'une déacétylation par hydrolyse acide à une température d'au moins 95°C pendant au moins 1 heure.

5. Procédé de synthèse selon l'une des revendications précédentes dans lequel la protection régiosélective de la fonction amine primaire en position 4 selon l'étape (b) comprend les étapes suivantes :
(b1) préparation d'un complexe de cuivre par mise en présence de l'acide 2,4-diamino-3-hydroxybutyrique obtenu à l'étape (a) avec un composé choisi parmi CuSO₄.5H₂O, CuSO₄, Cu₂(OH)₂CO₃, Cu(OAc)₂ et CuCO_{3;}
(b2) protection de la fonction amine primaire en position 4 par un groupement R₁ ;
(b3) éventuellement, décomplexation du cuivre pour obtenir le composé de formule suivante :

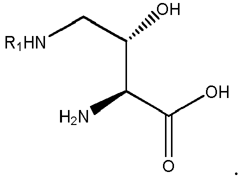

6. Procédé de synthèse selon la revendication 5 dans lequel l'étape (b2) consiste en la protection de la fonction amine primaire en position 4 par un groupement Boc.

7. Procédé de synthèse selon l'une des revendications précédentes dans lequel la protection de la fonction amine primaire en position 2 selon l'étape (c) est réalisée par réaction avec du FmocOSu.

8. Procédé de synthèse selon l'une des revendications précédentes dans lequel le composé de formule (I) a la formule suivante :

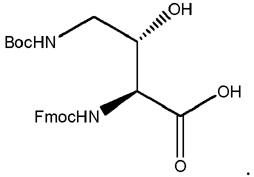

9. Procédé de synthèse selon l'une des revendications précédentes dans lequel l'étape (d) comprend la réaction de l'acide obtenu à l'étape (c) avec l'isopropylidène pour conduire au composé de formule (Id) suivante :

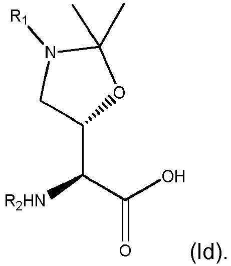

10. Procédé de synthèse selon l'une des revendications précédentes dans lequel le composé de formule (I) est obtenu avec une pureté diastéréoisomérique supérieure à 90%.

11. Procédé de synthèse de l'acide 2,4-diamino-3(S)-hydroxycarboxylique à partir de la 5-hydroxyectoïne.

12. Procédé de synthèse de l'acide 2,4-diamino-3(S)-hydroxycarboxylique selon la revendication 11 comprenant une hydrolyse basique de l'hydroxyectoïne et une déacétylation pour conduire à l'acide 2,4-diamino-3(S)-hydroxy butyrique.

## Patentansprüche

1. Verfahren zur Synthese von Verbindungen der folgenden Formel (I): 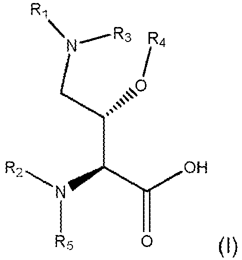 wobei:
- R₁ und R₂ unabhängig voneinander Schutzgruppen der Aminfunktionen bezeichnen;
- R₃ einen Wasserstoff oder eine Schutzgruppe der Aminfunktionen bezeichnet und R₄ einen Wasserstoff oder eine Schutzgruppe der Hydroxylfunktionen bezeichnet, oder R₁ und R₄ gemeinsam eine Gruppe bilden, die aus-C(CH₃)₂-, -CH(CH₃)-, -C((CH₂)₄)-, -C((CH₂)₅)-, -CH(C₆H₅)-, -CH((*p*-OCH₃)C₆H₄)-, -CH((*m,p*-OCH₃)C₆H₃)- und -C(O)-ausgewählt ist;
- R₅ einen Wasserstoff, ein Alkylradikal, ein Arylradikal oder ein Heteroarylradikal bezeichnet;
aus 5-Hydroxyectoin.

2. Syntheseverfahren nach Anspruch 1, umfassend die folgenden Schritte:
(a) basische Hydrolyse des Hydroxyectoins und Deacetylierung, um zu der 2,4-Diamino-3(S)-hydroxybuttersäure zu führen;
(b) regioselektiver Schutz durch eine Gruppe R₁ der primären Aminfunktion an Position 4 der in Schritt (a) erhaltenen 2,4-Diamino-3(S)-hydroxybuttersäure;
(c) Schutz durch eine Gruppe R₂ der primären Aminfunktion an Position 2 der in Schritt (b) erhaltenen Säure;
(d) eventuell Schutz der Hydroxylfunktion an Position 3 durch eine Gruppe R₄ und/oder Schutz des sekundären Amins an Position 4 durch eine Gruppe R₃ der in Schritt (c) erhaltenen Verbindung oder Schutz der Hydroxylfunktion an Position 3 und der sekundären Aminfunktion an Position 4, um eine Verbindung der Formel (I) zu erhalten, in welcher R₃ und R₄ gemeinsam eine Gruppe bilden, die aus -C(CH₃)₂-, -CH(CH₃)-,-C((CH₂)₄)-, -C((CH₂)₅)-, -CH(C₆H₅)-, -CH((*p*-OCH₃)C₆H₄)-,-CH((*m,p*-OCH₃)C₆H₃)- und -C(O)- ausgewählt ist;
(e) eventuell N-Alkylierung oder N-Arylierung an Position 2 der in Schritt (d) erhaltenen Verbindung, um eine Verbindung der Formel (I) zu erhalten, in welcher R₅ ein Alkylradikal, ein Arylradikal oder ein Heteroarylradikal ist;
(f) Rückgewinnung der Verbindung der Formel (I), erhalten in Schritt (c) oder gegebenenfalls in Schritt (d) oder (e).

3. Syntheseverfahren nach Anspruch 2, wobei die Hydrolyse und die Deacetylierung gemäß Schritt (a) in Gegenwart von mindestens 7 Moläquivalenten einer starken Base während mindestens 18 Stunden bei einer Temperatur von über oder gleich 85 °C durchgeführt werden.

4. Syntheseverfahren nach einem der vorangehenden Ansprüche, wobei die Hydrolyse gemäß Schritt (a) in Gegenwart von mindestens 1 Moläquivalent einer starken Base während mindestens 3 Stunden 30 bei einer Temperatur von über oder gleich 50 °C, gefolgt von einer Deacetylierung durch saure Hydrolyse bei einer Temperatur von mindestens 95 °C während mindestens 1 Stunde, durchgeführt wird.

5. Syntheseverfahren nach einem der vorangehenden Ansprüche, wobei der regioselektive Schutz der primären Aminfunktion an Position 4 gemäß Schritt (b) die folgenden Schritte umfasst:
(b1) Herstellung eines Kupferkomplexes durch Zusammenbringen der 2,4-Diamino-3(S)-hydroxybuttersäure aus Schritt (a) mit einer Verbindung, ausgewählt aus CuSO₄.5H₂O, CuSO₄, Cu₂(OH)₂CO₃, Cu(OAc)₂ und CuCO₃;
(b2) Schutz der primären Aminfunktion an Position 4 durch eine Gruppe R₁;
(b3) eventuell Dekomplexierung des Kupfers, um die Verbindung folgender Formel zu erhalten:

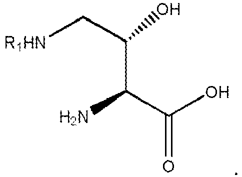

6. Syntheseverfahren nach Anspruch 5, wobei Schritt (b2) im Schutz der primären Aminfunktion an Position 4 durch eine Gruppe Boc besteht.

7. Syntheseverfahren nach einem der vorangehenden Ansprüche, wobei der Schutz der primären Aminfunktion an Position 2 gemäß Schritt (c) durch Reaktion mit FmocOSu durchgeführt wird.

8. Syntheseverfahren nach einem der vorangehenden Ansprüche, wobei die Verbindung der Formel (I) die folgende Formel hat:

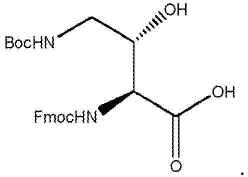

9. Syntheseverfahren nach einem der vorangehenden Ansprüche, wobei Schritt (d) die Reaktion der Säure aus Schritt (c) mit dem Isopropyliden umfasst, um zur Verbindung der folgenden Formel (Id) zu führen:

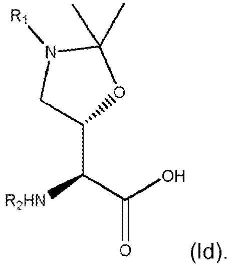

10. Syntheseverfahren nach einem der vorangehenden Ansprüche, wobei die Verbindung der Formel (I) mit einer Diastereoisomerreinheit von über 90 % erhalten wird.

11. Syntheseverfahren der 2,4-Diamino-3(S)-hydroxybuttersäure aus 5-Hydroxyectoin.

12. Syntheseverfahren der 2,4-Diamino-3(S)-hydroxybuttersäure nach Anspruch 11, umfassend eine basische Hydrolyse von Hydroxyectoin und eine Deacetylierung, um zur 2,4-Diamino-3(S)-hydroxybuttersäure zu führen.

## Claims

1. A process to synthesize compounds of following formula (I): 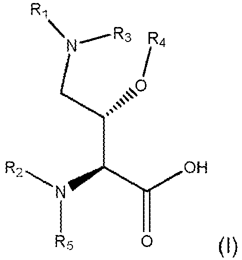 wherein:
- R₁ and R₂ independently of each other are protective groups of the amine functions;
- R₃ is a hydrogen or a protective group of the amine functions and R₄ is a hydrogen or a protective group of the hydroxyl functions, or R₃ and R₄ together form a group selected from among -C(CH₃)₂-, -CH(CH₃)-, -C((CH₂)₄)-, -C((CH₂)₅)-, -CH(C₆H₅)-, -CH((*p*-OCH₃,)C₆H₄)-, -CH((*m,p*-OCH₃)C₆H₃)- and -C(O)-;
- R₅ is a hydrogen, an alkyl radical, an aryl radical or an heteroaryl radical;
from 5-hydroxyectoine.

2. The synthetic process according to claim 1 comprising the following steps:
(a) alkaline hydrolysis of hydroxyectoine and deacetylation leading to 2,4-diamino-3(S)-hydroxy butyric acid;
(b) regioselective protection by a R₁ group of the primary amine function at position 4 of the 2,4-diamino-3(S)-hydroxybutyric acid obtained at step (a);
(c) protection by an R₂ group of the primary amine function at position 2 of the acid obtained at step (b) ;
(d) optionally, protection of the hydroxyl function at position 3 by an R₄ group and/or protection of the secondary amine at position 4 by an R₃ group of the compound obtained at step (c) or protection of the hydroxyl function at position 3 and secondary amine function at position 4 to obtain a compound of formula (I) wherein R₃ and R₄ together form a group selected from among -C(CH₃)₂-, -CH(CH₃)-, -C((CH₂)₄)-, -C((CH₂)₅)-, -CH(C₆H₅)-, -CH((*p*-OCH₃)C₆H₄)-, -CH((*m,p*-OCH₃)C₆H₃)- and -C(O)-;
(e) optionally, N-alkylation or N-arylation at position 2 of the compound obtained at step (d) to obtain a compound of formula (I) wherein R₅ is an alkyl radical, an aryl radical or a heteroaryl radical;
(f) recovering the compound of formula (I) obtained at step (c) or, when applicable, at step (d) or (e).

3. The synthesis process according to claim 2, wherein the hydrolysis and deacetylation at step (a) are performed in the presence of at least 7 molar equivalents of a strong base for at least 18hrs at a temperature of 85 °C or higher.

4. The synthesis process according to one of the preceding claims, wherein the hydrolysis at step (a) is performed in the presence of a least one molar equivalent of a strong base for at least 3h30 at a temperature of 50 °C or higher, followed by deacetylation via acid hydrolysis at a temperature of at least 95 °C for at least 1 hour.

5. The synthesis process according to one of the preceding claims, wherein the regioselective protection of the primary amine function at position 4 at step (b) comprises the following steps:
(b1) preparing a copper complex by placing the 2,4-diamino-3-hydroxybutyric acid obtained at step (a) in the presence of a compound selected from among CuSO₄.5H₂O, CuSO₄, Cu₂(OH)₂CO₃, Cu(OAc)₂ and CuCO₃;
(b2) protecting the primary amine function at position 4 by an R₁ group;
(b3) optionally, decomplexing the copper to obtain the compound of following formula:

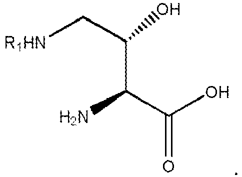

6. The synthesis process according to claim 5, wherein step (b2) consists of protecting the primary amine function at position 4 by a Boc group.

7. The synthesis process according to one of the preceding claims, wherein the protection of the primary amine function at position 2 at step (c) is performed by reaction with FmocOSu.

8. The synthesis process according to one of the preceding claims, wherein the formula (I) compound has the following formula:

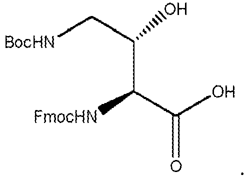

9. The synthesis process according to one of the preceding claims, wherein step (d) comprises the reaction of the acid obtained at step (c) with isopropylidene leading to the compound of following formula (Id) :

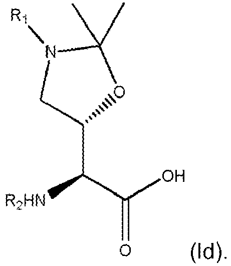

10. The synthesis process according to one of the preceding claims, wherein the formula (I) compound is obtained with a diastereoisomeric purity higher than 90%.

11. A process to synthesize 2,4-diamino-3(S)-hydroxycarboxylic acid from 5-hydroxyectoine.

12. The process to synthesize 2,4-diamino-3(S)-hydroxycarboxylic acid according to claim 11 comprising alkaline hydrolysis of hydroxyectoine and deacetylation to lead to 2,4-diamino-3(S)-hydroxy butyric acid.
